# EUROPEAN PATENT APPLICATION

(11) **EP 3 144 379 A1**
(43) Date of publication of application: **22.03.2017**
(21) Application number: 15793388.8
(22) Date of filing: 11.05.2015
(51) Int. Cl.: C12M 1/34

(54) **CULTURE OBSERVATION APPARATUS**

(30) Priority: 14.05.2014 JP 2014100575
(71) Applicant: Olympus Corporation, Tokyo 192-8507 (JP)
(72) Inventor: KAWANO, Yoshihiro, Tokyo 192-8507 (JP); KIMURA, Hiroyuki, Tokyo 192-8507 (JP)
(74) Representative: Gunzelmann, Rainer
(86) International application number: PCT/JP2015/063416
(87) International publication number: WO 2015/174356

(57) **Abstract**

Bothersomeness associated with a checking procedure during cell culturing is reduced.

Provided is a culturing observation apparatus (1) including: a light source portion (3) that radiates illumination light into a culturing container (C), in which cells are being cultured, from a side surface of the culturing container (C), which is optically transparent, within a predetermined angular range; an image-acquisition portion (4) that, when the illumination light radiated from the light source portion (3) is scattered at the cells in the culturing container C and when a portion of this scattered light passes through a bottom surface of the culturing container (C), acquires an image by capturing the scattered light that has passed through the bottom surface; and a transmitting portion (5) that transmits the image acquired by the image-acquisition portion 4 to an exterior.

## Description

### {Technical Field}

The present invention relates to a culturing observation apparatus.

### {Background Art}

In the related art, culturing cells involves repeating a procedure in which a culturing container is taken out of an incubator every time cells reach confluence, the cells are removed from the culturing container, and the cells are cultured by seeding the cells in a new culturing container (for example, see Patent Literature 1).

### {Citation List}

### {Patent Literature}

{PTL 1} Japanese Unexamined Patent Application, Publication No. Hei 6-217989

### {Summary of Invention}

### {Technical Problem}

However, this procedure is very bothersome because performing the procedure requires an observer to check the cells in the culturing container in the incubator once or twice a day.

The present invention has been conceived in light of the above-described circumstances, and an object thereof is to provide a culturing observation apparatus with which it is possible to reduce bothersomeness associated with the checking procedure during cell culturing.

### {Solution to Problem}

In order to achieve the above-described object, the present invention provides the following solutions.

An aspect of the present invention provides a culturing observation apparatus including: a light source portion that radiates illumination light into a culturing container from a side surface of the culturing container, which is transparent; an image-acquisition portion that acquires an image by capturing scattered light of the illumination light radiated from the light source portion, the scattered light coming from an interior of the culturing container; and a transmitting portion that transmits the image acquired by the image-acquisition portion to an exterior.

With the above-described aspect, when the illumination light emitted from the light source portion enters the culturing container from the side surface of the transparent culturing container in which the cells are being cultured, the illumination light is scattered at the cells, and, because this scattered light is emitted to the exterior from the culturing container, it is possible to detect the cell-culturing state in the interior of the culturing container by acquiring an image thereof by capturing the scattered light by means of the image-acquisition portion. Also, because the acquired image is transmitted to the exterior from the transmitting portion, for example, by receiving the transmitted image at the exterior of an incubator in which the culturing container is accommodated, it is possible to check the cell-culturing state in the interior of the culturing container without taking the culturing container out of the incubator by opening the lid thereof. Accordingly, it is possible to reduce bothersomeness during cell culturing.

In the above-described aspect, the light source portion may radiate the illumination light along an optical axis within a ± 30°-area with respect to the horizontal direction.

By doing so, the illumination light coming from the light source portion serves as dark-field illumination or oblique illumination, and thus, it is possible to form shadows of the cells being cultured in the culturing container. By doing so, the image-acquisition portion acquires a three-dimensional image of the cells, and it is possible to more clearly observe the cell-culturing state.

In addition, in the above-described aspect, the light source portion may make the illumination light enter at a height position between a bottom surface of the culturing container and a liquid surface of a culturing solution retained in the culturing container.

By doing so, because the illumination light does not pass through the liquid surface of the culturing solution and the bottom surface of the culturing container when the illumination light enters, the illumination light can reach a larger area in the culturing container.

In addition, in the above-described aspect, the image-acquisition portion may capture the scattered light that has passed through the bottom surface of the culturing container from the interior of the culturing container.

By doing so, in the case in which the cells being cultured in the culturing container are cells that grow while adhering to the bottom surface of the culturing container, it is possible to observe the cells from, via only the bottom surface of the culturing container, a position affected by the lowest number of obstacles. In addition, because sometimes droplets are formed on the top surface of the culturing container due to dew condensation caused by evaporation of the culturing solution, the droplets hinder observation; however, with observation via the bottom surface of the culturing container, such a problem does not occur.

In addition, in the above-described aspect, the image-acquisition portion may include: a focusing lens that is disposed so that an optical axis thereof is inclined with respect to the bottom surface of the culturing container; and an image-acquisition device that has an image-acquisition surface that is disposed so as to be inclined with respect to the optical axis of the focusing lens in a direction opposite from the bottom surface.

By doing so, it is possible to ensure a large enough distance between the focusing lens and the cells and to suppress the dimension in the height direction, and thus, it is possible to expand the observation area by reducing the magnification of the optical system and to make the apparatus more compact.

In addition, in the above-described aspect, the image-acquisition portion may include: a microlens array including a plurality of microlenses that are arrayed along the bottom surface of the culturing container; and an image-acquisition device that is disposed on the opposite side from the bottom surface of the culturing container, with the microlens array interposed therebetween.

By doing so, it is possible to acquire a clear image by forming an image of cells in the image-acquisition device by using the microlenses. It is possible to set the focal distances of the microlenses to be sufficiently small, and thus, it is possible to configure the apparatus in a compact manner by suppressing the dimension thereof in the height direction.

In addition, in the above-described aspect, a plurality of the culturing containers may be arranged in a stacked state, and the image-acquisition portion may be disposed farther out from the side surface of the culturing container, and a moving mechanism that moves the image-acquisition portion in a top-to-bottom direction may be provided.

By doing so, it is possible to monitor the culturing state by moving the image-acquisition portion in the top-to-bottom direction by operating the moving mechanism and by capturing, by means of the image-acquisition portion, the scattered light that has passed through the bottom surface from the interior of the culturing container to be observed. In the case in which the plurality of culturing containers arranged in the stacked state are accommodated in the incubator and a large amount of cells are cultured at a time, it is possible to efficiently monitor the cell-culturing states in the individual culturing containers.

In addition, in the above-described aspect, a plurality of the culturing containers may be arranged in a stacked state, and the above-described aspect may include: an observation optical system that has an optical axis that is parallel to a direction in which the culturing containers are stacked, that is disposed above or below the culturing containers, and in which a focal distance thereof can be adjusted; and an image-acquisition device that captures scattered light coming from a specimen disposed at a focal position of the observation optical system.

By doing so, by aligning the cells in the culturing container to be observed with the focal position by adjusting the focal distance of the observation optical system, it is possible to acquire a clear image by using the image-acquisition device. In the case in which the plurality of culturing containers arranged in the stacked state are accommodated in the incubator and a large amount of cells are cultured at a time, it is possible to efficiently monitor the cell-culturing states in the individual culturing containers.

In addition, in the above-described aspect, the light source portion may be disposed so as to face side surfaces of the individual culturing containers, and the above-described aspect may include a light-source control portion that selectively causes the light source portion corresponding to the specimen disposed at the focal position of the objective optical system of the image-acquisition portion to emit illumination light.

By doing so, it is possible to capture an image by radiating the illumination light only onto the cells that are aligned with the focal position of the observation optical system, and it is possible to acquire a highly visible image by preventing flare formation in the acquired image by not irradiating the rest of the culturing containers with the illumination light.

### {Advantageous Effects of Invention}

The present invention affords an advantage in that it is possible to reduce bothersomeness associated with a checking procedure during cell culturing.

### {Brief Description of Drawings}

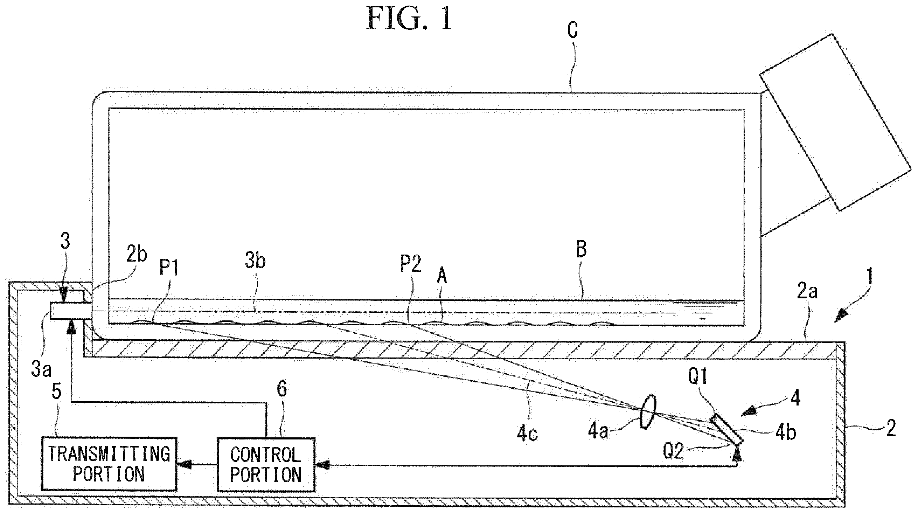

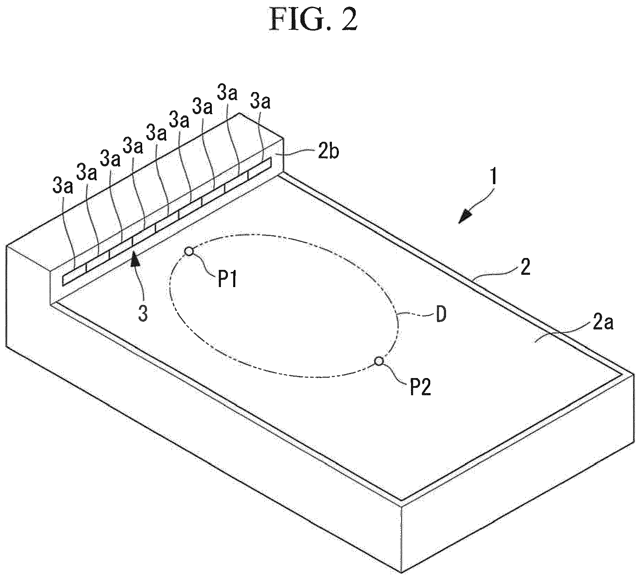

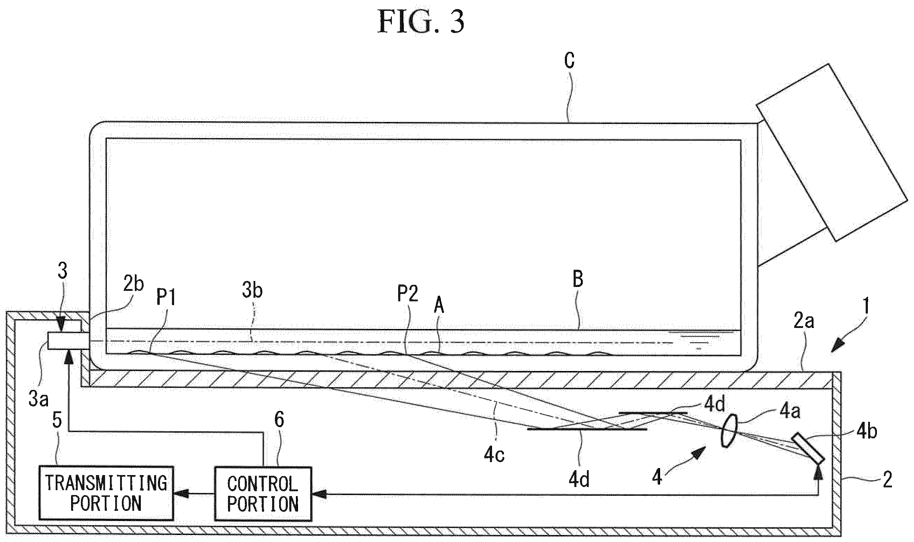

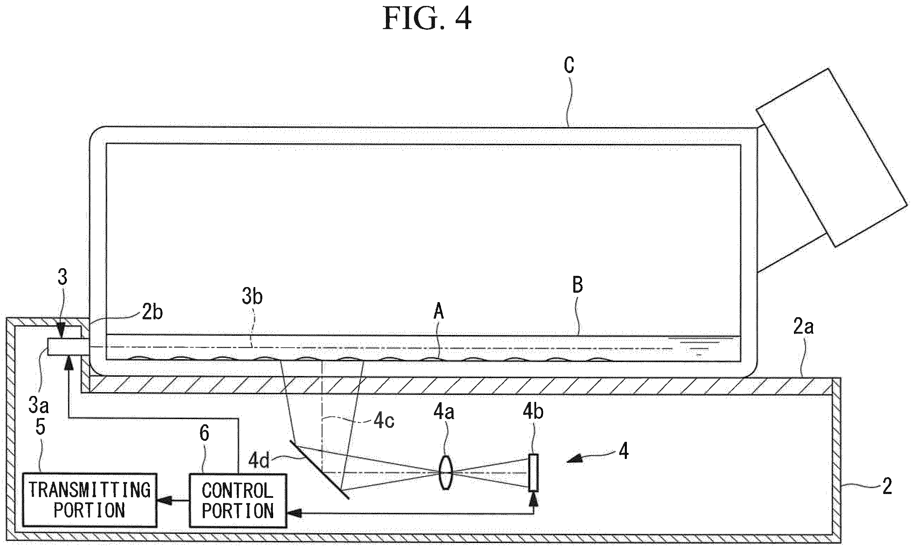

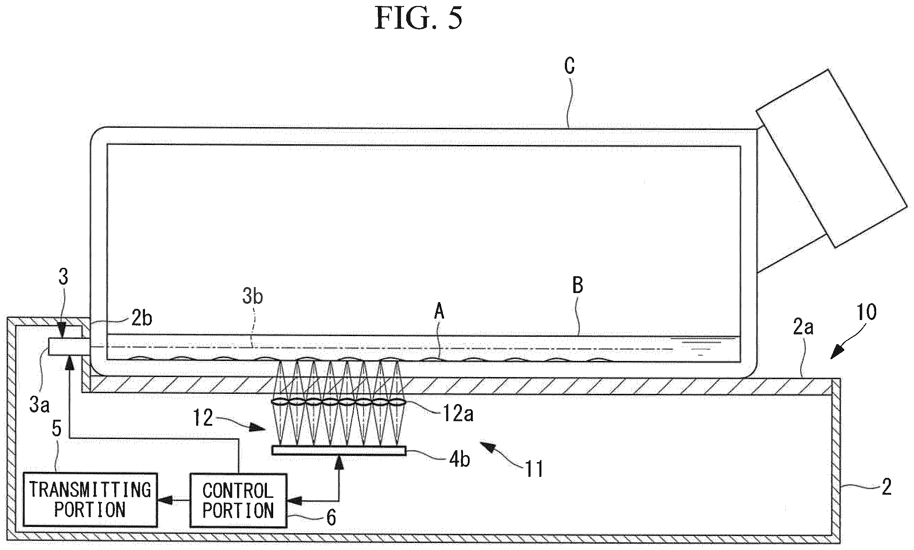

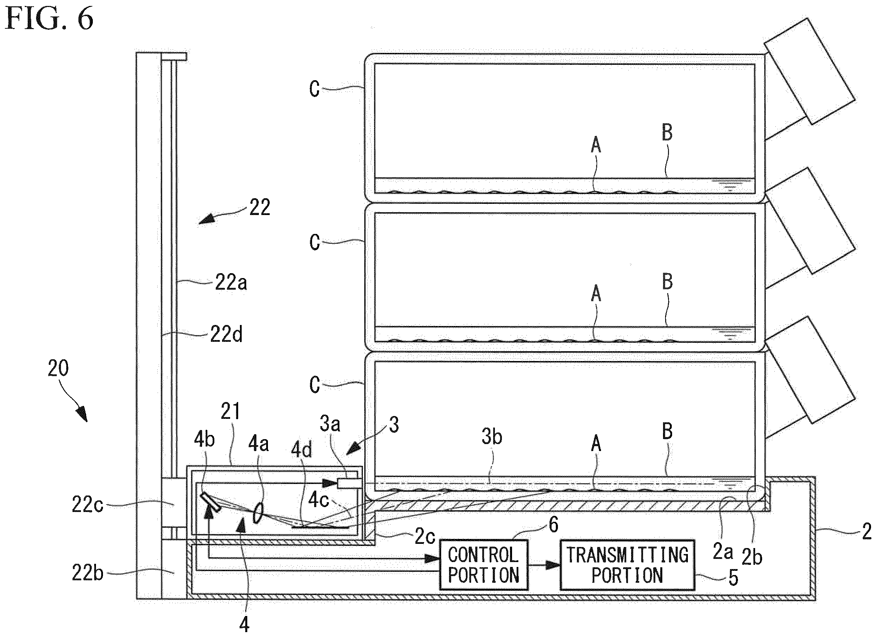

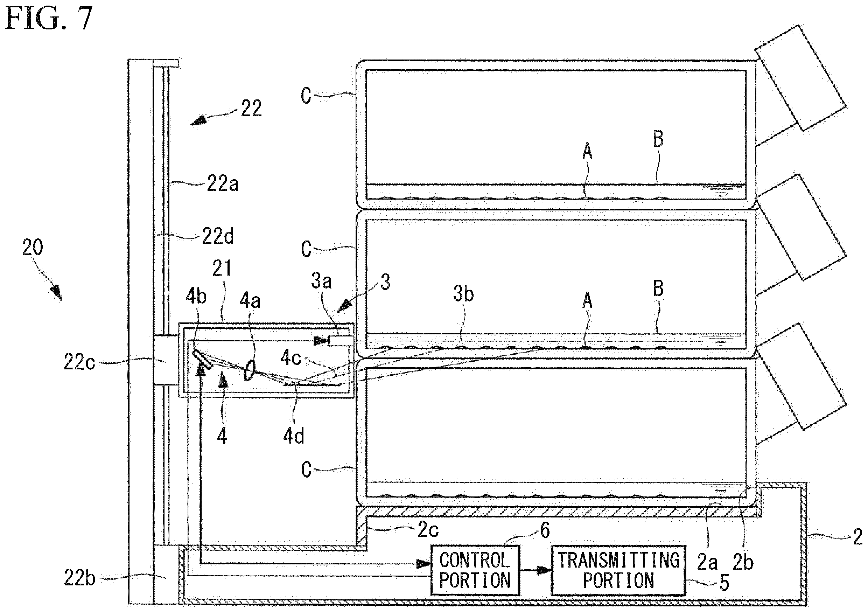

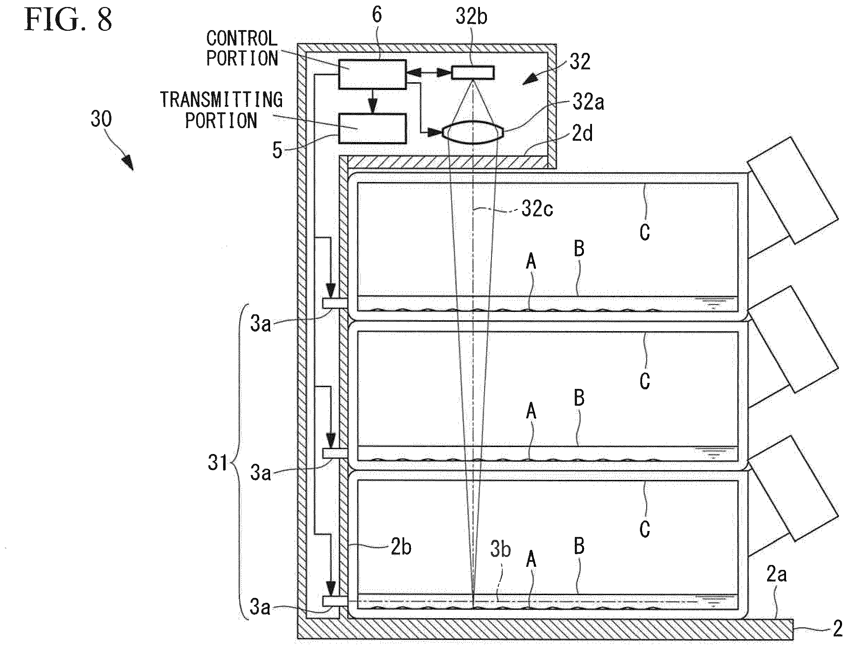

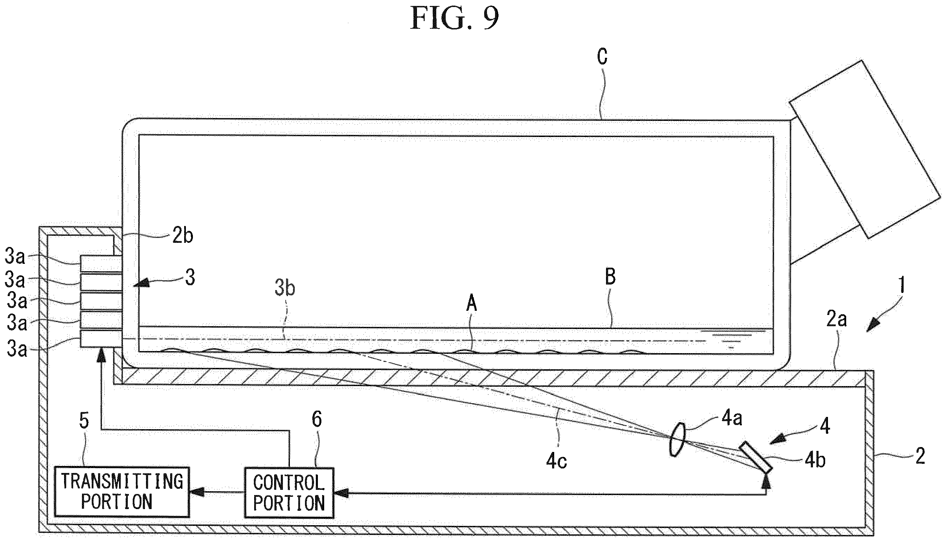

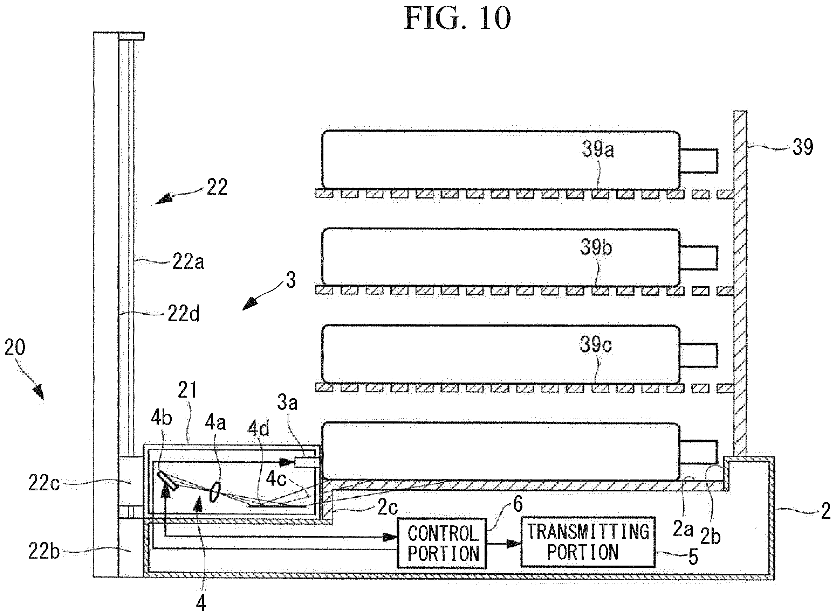

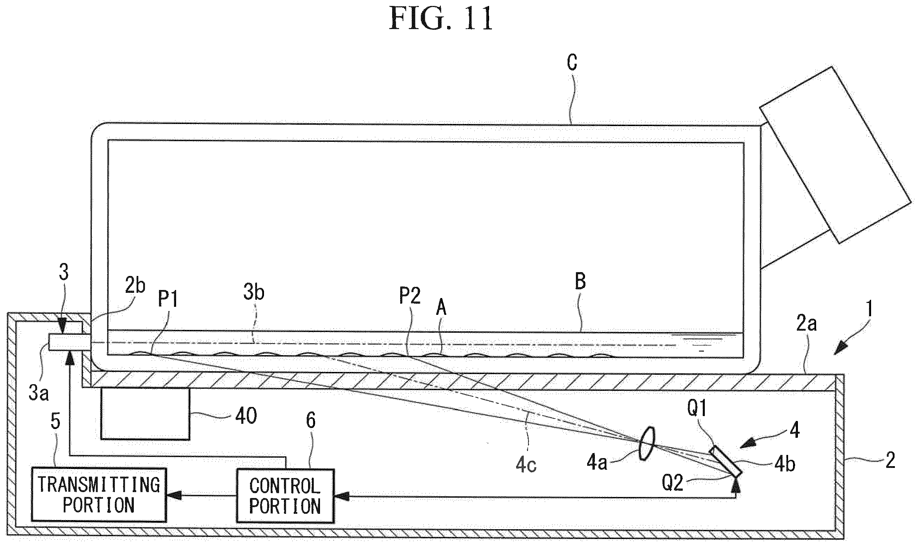

{Fig. 1} Fig. 1 is a longitudinal cross-sectional view showing a culturing observation apparatus according to a first embodiment of the present invention.
{Fig. 2} Fig. 2 is a perspective view showing the culturing observation apparatus in Fig. 1.
{Fig. 3} Fig. 3 is a longitudinal cross-sectional view showing a modification of the culturing observation apparatus in Fig. 1.
{Fig. 4} Fig. 4 is a longitudinal cross-sectional view showing another modification of the culturing observation apparatus in Fig. 1.
{Fig. 5} Fig. 5 is a longitudinal cross-sectional view showing a culturing observation apparatus according to a second embodiment of the present invention.
{Fig. 6} Fig. 6 is a longitudinal cross-sectional view showing a culturing observation apparatus according to a third embodiment of the present invention.
{Fig. 7} Fig. 7 is a longitudinal cross-sectional view showing a state in which a movable portion of the culturing observation apparatus in Fig. 6 is raised.
{Fig. 8} Fig. 8 is a longitudinal cross-sectional view showing a culturing observation apparatus according to a fourth embodiment of the present invention.
{Fig. 9} Fig. 9 is a longitudinal cross-sectional view showing another modification of the culturing observation apparatus in Fig. 1.
{Fig. 10} Fig. 10 is a longitudinal cross-sectional view showing another modification of the culturing observation apparatus in Fig. 6.
{Fig. 11} Fig. 11 is a longitudinal cross-sectional view showing another modification of the culturing observation apparatus in Fig. 1.

### {Description of Embodiments}

A culturing observation apparatus 1 according to a first embodiment of the present invention will be described below with reference to the drawings.

As shown in Fig. 1, the culturing observation apparatus 1 according to this embodiment includes: a base 2 on which a culturing container C that accommodates cells A to be cultured together with a culturing solution B is installed; and a light source portion 3, an image-acquisition portion 4, a transmitting portion 5, and a control portion 6 that are provided in the base 2.

The culturing container C is, for example, a cell-culturing flask, and is formed of an optically transparent material.

As shown in Figs. 1 and 2, the base 2 includes: an installing surface 2a that is formed of an optically transparent material and with which a lower surface of the culturing container C is brought into a close contact; and an abutting surface 2b that is provided upright on the installing surface 2a and with which one side surface of the culturing container C installed on the installing surface 2a is brought into close contact. Because the incubator interior is in a high-humidity state, the base 2 has a waterproofed structure.

The light source portion 3 includes a plurality of LED light sources 3a that are disposed at the abutting surface 2b, and that are arrayed in a direction parallel to the installing surface 2a with a predetermined space from the installing surface 2a. The predetermined space from the installing surface 2a is set to be equal to or slightly greater than a distance from a lower surface of the culturing container C to be installed to the bottom surface of the interior of the culturing container C and to be smaller than the distance to the liquid surface of the culturing solution B expected to be retained in the culturing container C.

In addition, optical axes 3b of the illumination light beams emitted from the individual LED light sources 3a are set so as to be substantially parallel to the installing surface 2a.

The image-acquisition portion 4 includes: a focusing lens 4a that is disposed below the installing surface 2a in the interior of the base 2; and an image-acquisition device 4b that acquires an image by capturing light focused by the focusing lens 4a.

The focusing lens 4a is disposed so that an optical axis 4c is inclined with respect to the installing surface 2a and so that the optical axis 4c intersects the installing surface 2a and the bottom surface of the culturing container C installed on the installing surface 2a.

The image-acquisition device 4b is also disposed on the opposite side from the installing surface 2a with the focusing lens 4a interposed therebetween and is disposed on the optical axis 4c of the focusing lens 4a. As shown in Fig. 1, the image-acquisition device 4b has an image-acquisition surface that is inclined with respect to the optical axis 4c of the focusing lens 4a in a direction opposite from the direction in which the installing surface 2a is inclined. By doing so, light coming from a position P1 away from the focusing lens 4a forms an image on the image-acquisition surface at a position Q1 close to the focusing lens 4a, whereas light coming from a position P2 close to the focusing lens 4a forms an image on the image-acquisition surface at a position Q2 away from the focusing lens 4a. Therefore, it is possible to make the light, which is coming from the bottom surface of the culturing container C installed on the installing surface 2a inclined with respect to the optical axis 4c of the focusing lens 4a, form an image on the image-acquisition surface of the image-acquisition device 4b over a large area.

The transmitting portion 5 is configured so as to wirelessly transmit an image acquired by the image-acquisition device 4b to the outside.

In addition, the control portion 6 includes, for example, a timer (not shown), and is configured so as to periodically operate the light source portion 3, the image-acquisition portion 4, and the transmitting portion 5.

The operation of the thus-configured culturing observation apparatus 1 according to this embodiment will be described below.

In order to observe the culturing state of the cells A by using the culturing observation apparatus 1 according to this embodiment, the culturing container C, in which the cells A to be cultured and the culturing solution B are accommodated, is installed on the base 2 so that the lower surface thereof comes into close contact with the installing surface 2a of the base 2 and so that the one side surface thereof is abutted to the abutting surface 2b of the base 2.

In this state, the culturing observation apparatus 1 in which the culturing container C is installed is accommodated in the incubator (not shown), and the culturing observation apparatus 1 is disposed so that the installing surface 2a is horizontally set, thus starting culturing of the cells A in the culturing container C in an environment in which the temperature and humidity in the incubator are managed. In addition, the timer in the control portion 6 is activated at this point, and the clock is started.

Once culturing is started, a schedule that is set in advance in accordance with the clock result of the timer is followed, thus operating the light source portion 3 by means of the control portion 6, turning on the LED light sources 3a, and capturing an image by using the image-acquisition device 4b.

The LED light sources 3a are provided at the abutting surface 2b to which the side surface of the culturing container C is abutted, and make the illumination light enter the culturing container C from the side surface of the culturing container C in the direction of the optical axes 3b, which are parallel to the bottom surface of the culturing container C. By doing so, as with oblique illumination or dark-field illumination, the cells A that are growing while being adhered to the bottom surface of the culturing container C are laterally irradiated, thus forming shadows of the cells A.

With the scattered light that has been scattered at the cells A, a portion thereof passes through the bottom surface of the culturing container C and the installing surface 2a of the base 2, is focused by the focusing lens 4a in the base 2, and is captured by the image-acquisition device 4b. In this case, with the culturing observation apparatus 1 according to this embodiment, because the focusing lens 4a is disposed so that the optical axis 4c is inclined with respect to the installing surface 2a, scattered light coming from an area D in which the optical axis 4c is at the center thereof and that extends so as to be elongated in the direction in which the optical axis 4c is inclined forms an image in the image-acquisition device 4b.

In this embodiment, because the image-acquisition device 4b is disposed so as to be inclined in the direction opposite from the installing surface 2a, as shown in Fig. 1, in the bottom surface of the culturing container C, scattered light coming from a portion located at the position P1 far from the focusing lens 4a forms an image in the image-acquisition device 4b disposed at the position Q1 close to the focusing lens 4a, and scattered light coming from a portion located at the position P2 close to the focusing lens 4a forms an image in the image-acquisition device 4b disposed at the position Q2 far from the focusing lens 4a.

As a result, a focused image of the bottom surface of the culturing container C is formed in the image-acquisition device 4b over a large area, and thus, it is possible to acquire an image of the bottom surface of the culturing container C over a large area. In addition, because the optical axis 4c of the focusing lens 4a is inclined with respect to the bottom surface of the culturing container C, there is an advantage in that, while suppressing the thickness of the space below the installing surface 2a in which the focusing lens 4a and the image-acquisition device 4b are accommodated, it is possible to ensure a large enough distance, parallel to the optical axis 4c, between the focusing lens 4a and the installing surface 2a and to capture an image of the bottom surface of the culturing container C over a large area at a low magnification.

After the image is acquired, the LED light sources 3a are turned off each time. By intermittently operating the light source portion 3 or the like in this way, it is possible to reduce the influence of heat on the cells by suppressing an increase in the temperature of the apparatus.

Then, the image acquired by the image-acquisition device 4b is transmitted to the transmitting portion 5 from the control portion 6, thus being externally transmitted by the transmitting portion 5. Therefore, by receiving the image that has been transmitted from the transmitting portion 5 at the exterior of the incubator and by displaying the image on the monitor, it is possible to check, at the exterior of the incubator, the culturing state of the cells A in the culturing container C without having to observe the culturing container C by taking the culturing container C out of the incubator and, additionally, without having to open the incubator door. In other words, there is an advantage in that it is possible to considerably reduce the bothersomeness of the checking procedure during cell culturing. In addition, because it is not necessary to take the culturing container C out of the incubator, it is possible to eliminate environmental changes (changes in the temperature, the pH, or the like) to which the cells may be exposed.

In particular, with this embodiment, because the light source portion 3 makes the illumination light enter the culturing container C so as to be parallel to the bottom surface of the culturing container C to which the cells A are adhered, it is possible to form shadows of the cells A being cultured in the culturing container C. By doing so, the image-acquisition portion 4 acquires a three-dimensional image of the cells A, and thus, it is possible to more clearly observe the culturing state of the cells A.

In addition, because the light source portion 3 makes the illumination light enter between the bottom surface of the culturing container C and the liquid surface of the culturing solution B, the illumination light need not pass through the liquid surface of the culturing solution B and the bottom surface of the culturing container C, and thus, it is possible to propagate the illumination light farther in the culturing solution B by suppressing scattering thereof, and it is possible to illuminate a larger area.

In addition, because the image-acquisition portion 4 captures, of the scattered light at the cells A being cultured, the scattered light that has passed through the bottom surface of the culturing container C, it is possible to acquire a clear image without being affected by the influence of droplets formed by dew condensation on the top surface of the culturing container C in the case in which culturing is performed in a high-temperature, high-humidity environment.

In addition, in this embodiment, because the LED light sources 3a are employed as the light source portion 3, there is an advantage in that it is possible to suppress power consumption by suppressing heat generation, thus reducing the influence on the cells.

Note that, in this embodiment, as shown in Fig. 3, the optical axis 4c of the focusing lens 4a may be folded by one or more mirrors 4d. By doing so, there is an advantage in that, while suppressing the thickness of the base 2 further, it is possible to ensure a large enough distance between the focusing lens 4a and the installing surface 2a, and it is possible to capture an image of the bottom surface of the culturing container C at a low magnification over a large area. By reducing the thickness of the base 2, it is possible to reduce the space when accommodating the culturing container C in the incubator, and thus, it is effective in the case in which it is necessary to accommodate many culturing containers C in the incubator at a time.

In addition, in this embodiment, although the illumination light coming from the light source portion 3 is made to enter the culturing container C in the horizontal direction along the optical axes 3b, which are parallel to the bottom surface thereof, it is not limited thereto, and the illumination light may be made to enter at an angle equal to or less than ± 30° with respect to the horizontal direction. By using such an angle also, it is possible to form shadows of the cells A that are the same as the case where dark-field illumination or oblique illumination is employed, and thus, it is possible to capture a three-dimensional image.

In addition, in this embodiment, although an image of the bottom surface of the culturing container C is acquired over a large area by making the optical axis 4c of the focusing lens 4a inclined with respect to the bottom surface of the culturing container C, alternatively, as shown in Fig. 4, a partial image of the bottom surface may be acquired by making the optical axis 4c of the focusing lens 4a orthogonal to the bottom surface. In this case, as compared with the case in which an image of the entire bottom surface or a relatively large portion of the bottom surface is captured, although the reliability of judging the culturing state decreases, the culturing state may be estimated on the basis of the image of the area of this portion.

In addition, in this embodiment, although the control portion 6 includes the timer, and the light source portion 3 or the like is periodically operated, alternatively, a receiving portion (not shown) may be connected to the control portion 6, instruction signals may be received from the exterior of the incubator, and the control portion 6 may drive the light source portion 3 or the like in accordance with the instruction signals. By doing so, it is possible for an operator to turn the light source portion 3 on and off and to capture images at an arbitrary timing by means of remote operation.

Transmitting and receiving of the image signals and the instruction signals may be performed by means of wireless or wired communication.

Next, a culturing observation apparatus 10 according to a second embodiment of the present invention will be described below with reference to the drawings.

In describing this embodiment, portions having the same configurations as those in the above-described culturing observation apparatus 1 according to the first embodiment are given the same reference signs, and descriptions thereof will be omitted.

The culturing observation apparatus 10 according to this embodiment differs from the culturing observation apparatus 1 according to the first embodiment in terms of an image-acquisition portion 11.

As shown in Fig. 5, the image-acquisition portion 11 in this embodiment includes: a microlens array 12 including, below the installing surface 2a, a plurality of microlenses 12a that are arrayed in a plane that is substantially parallel to the installing surface 2a; and the image-acquisition device 4b that is disposed farther below the microlens array 12. The microlenses 12a of the microlens array 12 are disposed so as to correspond to each one of pixels of the image-acquisition device 4b.

The focal distances of the individual microlenses 12a are set to be greater than a thickness obtained by adding the thickness of the transparent member that forms the installing surface 2a and the thickness of the bottom surface of the culturing container C installed on the installing surface 2a, and, by disposing the focal position below the installing surface 2a so as to be aligned with the cells A that are adhered to the bottom surface of the culturing container C, it is possible to project the image of the cells A on the image-acquisition surface of the image-acquisition device 4b.

Note that, it is not necessarily required for the image-acquisition device 4b to capture an image of the whole bottom surface of the culturing container C, and the culturing state may be estimated on the basis of an acquired image by acquiring an image of an area at an arbitrary portion, such as a center portion or the like, in which the probability that the cells A exist is high.

Next, a culturing observation apparatus 20 according to a third embodiment of the present invention will be described below with reference to the drawings.

In describing this embodiment, portions having the same configurations as those in the above-described culturing observation apparatus 1 according to the first embodiment are given the same reference signs, and descriptions thereof will be omitted.

As shown in Figs. 6 and 7, the culturing observation apparatus 20 according to this embodiment is a culturing observation apparatus 20 that is used in the case in which a plurality of culturing containers C are arranged in a stacked state and are accommodated in an incubator, and includes: a base 2 in which the position thereof is set with respect to the culturing containers C; a movable portion 21 that is provided so as to be movable in the top-to-bottom direction with respect to the base 2; and a moving mechanism 22 that moves the movable portion 21 relative to the base 2.

The base 2 includes: for example, the installing surface 2a with which the lower surface of the culturing container C at the lowest position among the plurality of culturing containers C in a stacked state is brought into close contact; and the abutting surface 2b to which the side surface of that culturing container C is abutted.

The installing surface 2a of the base 2 and a side surface 2c that is a portion thereof below the installing surface 2a are formed of an optically transparent material, and are configured so that scattered light coming from the bottom surface of the culturing container C installed on the installing surface 2a can be observed from the exterior via the installing surface 2a and the side surface 2c.

The movable portion 21 supports the light source portion 3 that emits the illumination light and the image-acquisition portion 4 in a state in which the relative positions thereof are set.

As with the first embodiment, the light source portion 3 includes the LED light sources 3a, is disposed so as to face the side surface of the culturing container C, and is configured so as to irradiate the interior of the culturing container C with the illumination light in a substantially horizontal direction by passing through the side surface of the culturing container C.

As with the first embodiment, the image-acquisition portion 4 includes: the focusing lens 4a having the optical axis 4c inclined with respect to the bottom surface of the culturing container; and the image-acquisition device 4b that captures the scattered light focused by the focusing lens 4a, wherein the image-acquisition device 4b is disposed so as to be inclined with respect to the optical axis 4c of the focusing lens 4a in a direction opposite from the bottom surface of the culturing container C. The image-acquisition portion 4 is configured so as to be disposed diagonally below the culturing container C being irradiated with the illumination light coming from the LED light sources 3a when the LED light sources 3a constituting the light source portion 3 are disposed at a height position between the bottom surface of any one of the culturing containers C and the liquid surface of the culturing solution B retained in that culturing container C, and thus the image-acquisition portion 4 is configured so as to be disposed at a position for capturing an image of the cells A being cultured in that culturing container C.

By doing so, of the scattered light at the cells A adhered to the bottom surface of the culturing container C, the scattered light that has passed through the bottom surface of the culturing container C and the top surface and the side surface of the culturing container C therebelow or the bottom surface of the culturing container C and the installing surface 2a and the side surface 2c of the base 2 can be focused by the focusing lens 4a and can be captured by the image-acquisition device 4b.

As the moving mechanism 22, it is possible to employ, among others, a mechanism which includes: for example, a ball screw 22a; a motor 22b that rotates the ball screw 22a about an axis; a nut 22c that is secured to the movable portion 21 and that engages with the ball screw 22a; and a guide rail 22d that supports the movable portion 21 so as to be movable in the top-to-bottom direction.

The control portion 6 is periodically driven on the basis of the timer, and is configured so as to capture, when being driven, images of the culturing states of the cells A being cultured in the individual culturing containers C by repeatedly performing irradiation of the illumination light from the light source portion 3, capturing of the images by the image-acquisition portion 4, and raising/lowering operations of the movable portion 21 by the moving mechanism 22.

The raising/lowering operations of the movable portion 21 by the moving mechanism 22 are intermittently performed in an amount corresponding to the thickness of the culturing container C.

With the thus-configured culturing observation apparatus 20 according to this embodiment, the control portion 6 operates the light source portion 3; causes the image-acquisition portion 4 to capture an image of the bottom surface of the culturing container C, the interior of which the illumination light has entered; causes, after the image has been captured, the movable portion 21 to be raised/lowered by operating the moving mechanism 22, as shown in Fig. 7; and repeats the operations of the light source portion 3 and the image-acquisition portion 4; and thus, it is possible to sequentially observe the culturing states of the cells A in all culturing containers C in the stacked state. Therefore, there is an advantage in that it is possible to observe the culturing states of the cells A in the individual culturing containers C from the exterior of the incubator even in the case in which culturing is performed by accommodating many culturing containers C in the incubator at a time.

Note that, in the above-described individual embodiments, although a focused image is acquired over a large area by making the image-acquisition device 4b inclined with respect to the optical axis 4c of the focusing lens 4a, a microlens array including a plurality of microlenses (not shown) having different focal distances may be employed instead of the single focusing lens 4a. By doing so, even if the image-acquisition device 4b is disposed orthogonal to the optical axis of the microlens array, it is possible to acquire a focused image over a large area.

Next, a culturing observation apparatus 30 according to a fourth embodiment of the present invention will be described below with reference to the drawings.

In describing this embodiment, portions having the same configurations as those in the above-described culturing observation apparatus 1 according to the first embodiment are given the same reference signs, and descriptions thereof will be omitted.

As shown in Fig. 8, the culturing observation apparatus 30 according to this embodiment differs from the culturing observation apparatus 1 according to the first embodiment in that it is provided with a light source portion 31, in which multiple levels of the LED light sources 3a are arranged in the top-to-bottom direction with spaces therebetween at the abutting portion 2b, and an image-acquisition portion 32, disposed above the culturing containers C arranged in the stacked state.

The size of the spaces, in the top-to-bottom direction, between the LED light sources 3a of light source portion 31 in this embodiment matches with the height of the culturing container C. The LED light source 3a at the lowest level is disposed at, as with the first embodiment, a position at which it is possible to make the illumination light enter the culturing container C at the lowest level from a height between the installing surface 2a of the base 2 on which the culturing containers C are installed and the liquid surface of the culturing solution B retained in the culturing container C at the lowest level. Therefore, it is possible for all of the LED light sources 3a to make the illumination light enter corresponding culturing containers C from heights between the bottom surfaces of the corresponding culturing containers C and the liquid surfaces of the culturing solutions B.

The image-acquisition portion 32 includes: a focusing lens 32a that is disposed so that an optical axis 32c thereof points in the vertical direction; and an image-acquisition device 32b that captures light focused by the focusing lens 32a. The focusing lens 32a is a variable-focus lens in which the focal distance thereof can be changed. The variable-focus lens may be one employing a system in which lenses are switched or may be one in which the focal distance thereof can be changed by applying a voltage thereto, as with a liquid lens.

In addition, in this embodiment, when the control portion 6 selects an LED light source 3a from which the illumination light is emitted, the control portion 6 controls the focusing lens 32a so that the focal point of the focusing lens 32a is aligned with the bottom-surface position of a culturing container C at which that LED light source 3a is disposed so as to face the culturing container C.

In the figure, the reference sign 2d is a wall surface formed of an optically transparent material.

With the thus-configured culturing observation apparatus 30 according to this embodiment, when the illumination light is made to enter a culturing container C from any one of the LED light sources 3a selected by the control portion 6 (one at the lowest position in the example shown in Fig. 8), because the focal position of the focal lens 32a is aligned with the bottom surface of the culturing container C (at the lowest position) corresponding to the LED light source 3a, it is possible to acquire an image of the cells A being cultured on the bottom surface of the culturing container C. After acquiring the image, by changing the LED light source 3a from which the illumination light is emitted and by changing the focal position of the focusing lens 32a, it is possible to sequentially acquire images of the cells A in the culturing containers C in the stacked state.

In this case, because only the LED light source 3a corresponding to the culturing container C in which an image is to be captured is operated, and illumination light is not emitted from the rest of the LED light sources 3a, it is possible to acquire a clear image by preventing illumination light coming from the rest of the LED light sources 3a from appearing in the image in the form of flare.

Note that, in this embodiment, although images of the bottom surfaces of the plurality of culturing containers C are acquired from above by using the image-acquisition portion 32 disposed above the culturing containers C, alternatively, images of the bottom surfaces of the plurality of culturing containers C may be captured from below by disposing the image-acquisition portion 32 below the installing surface 2a and by changing the focal distance. In addition, in the case in which the culturing containers C are stacked in a large number, because images become less clear with an increase in the focal distance, the focal distance may be reduced by disposing the image-acquisition portions 32 both above and below.

In addition, because the distance from the lower surface to the bottom surface is different depending on the type of the culturing container C, as shown in Fig. 9, multiple levels of the LED light sources 3a may be arranged in the top-to-bottom direction, and the LED light sources 3a to be turned on may be selected in accordance with the type of the culturing container C.

As in the culturing observation apparatus according to the third embodiment or the fourth embodiment, described above, in the form in which a plurality of culturing containers are arranged in a stacked state, in the case in which cell-culturing bags are employed as the culturing containers, for example, it is possible to provide a holding-rack unit 39 having a plurality of holding racks 39a, 39b, and 39c, as shown in Fig. 10. By doing so, it is possible to dispose the plurality of cell-culturing bags in the vertical direction. Here, the holding racks have a structure that does not hinder image acquisition by the image-acquisition portion, and the holding racks may be formed of, for example, an optically transparent material, or may have a structure in which an opening is provided in the observation area.

As shown in Fig. 11, in the culturing observation apparatuses according to the above-described individual examples, it is possible to provide a vibrating means 40 for causing an installed culturing container to vibrate. By doing so, in the case in which the cells in the culturing container are adherent cells, when performing cell-removal treatment such as trypsin treatment or the like, it is possible to facilitate the cell removal by causing the culturing container to vibrate by using the vibrating means 40, and, in addition, it is possible to observe whether or not the cells have been removed. The vibrating means 40 may be controlled by the control portion.

### {Reference Signs List}

- B: culturing solution
- C: culturing container
- 1, 10, 20, 30: culturing observation apparatus
- 3: light source portion
- 3b: optical axis
- 4, 11, 32: image-acquisition portion
- 4a: focusing lens
- 4b: image-acquisition device
- 4c: optical axis
- 5: transmitting portion
- 6: control portion (light-source control portion)
- 12: microlens array
- 12a: microlens
- 22: moving mechanism
- 32a: focusing lens (observation optical system)

## Claims

1. A culturing observation apparatus comprising:
a light source portion that radiates illumination light into a culturing container from a side surface of the culturing container, which is transparent;
an image-acquisition portion that acquires an image by capturing scattered light of the illumination light radiated from the light source portion, the scattered light coming from an interior of the culturing container; and
a transmitting portion that transmits the image acquired by the image-acquisition portion to an exterior.

2. A culturing observation apparatus according to Claim 1, wherein the light source portion radiates the illumination light along optical axes within a ±30°-area with respect to the horizontal direction.

3. A culturing observation apparatus according to Claim 1 or 2, wherein the light source portion makes the illumination light enter at a height position between a bottom surface of the culturing container and a liquid surface of a culturing solution retained in the culturing container.

4. A culturing observation apparatus according to any one of Claims 1 to 3, wherein the image-acquisition portion captures the scattered light that has passed through the bottom surface of the culturing container from the interior of the culturing container.

5. A culturing observation apparatus according to Claim 4,
wherein the image-acquisition portion comprises:
a focusing lens that is disposed so that an optical axis thereof is inclined with respect to the bottom surface of the culturing container; and
an image-acquisition device that has an image-acquisition surface that is disposed so as to be inclined with respect to the optical axis of the focusing lens in a direction opposite from the bottom surface.

6. A culturing observation apparatus according to Claim 4,
wherein the image-acquisition portion comprises:
a microlens array including a plurality of microlenses that are arrayed along the bottom surface of the culturing container; and
an image-acquisition device that is disposed on the opposite side from the bottom surface of the culturing container, with the microlens array interposed therebetween.

7. A culturing observation apparatus according to Claim 5,
wherein a plurality of the culturing containers are arranged in a stacked state, and
the image-acquisition portion is disposed farther out from the side surface of the culturing container,
the culturing observation apparatus further comprising:
a moving mechanism that moves the image-acquisition portion in a top-to-bottom direction.

8. A culturing observation apparatus according to any one of Claims 1 to 3,
wherein a plurality of the culturing containers are arranged in a stacked state, and
the image-acquisition portion comprises:
an observation optical system that has an optical axis that is parallel to a direction in which the culturing containers are stacked, that is disposed above or below the culturing containers, and in which a focal distance thereof can be adjusted; and
an image-acquisition device that captures scattered light coming from a specimen disposed at a focal position of the observation optical system.

9. A culturing observation apparatus according to Claim 8,
wherein the light source portion is disposed so as to face side surfaces of the individual culturing containers, the culturing observation apparatus further comprising:
a light-source control portion that selectively causes the light source portion corresponding to the specimen disposed at the focal position of the objective optical system of the image-acquisition portion to emit illumination light.
